(19) **Europäisches Patentamt European Patent Office Office européen des brevets**

(11) **EP 3 614 140 B1**

(12) **FASCICULE DE BREVET EUROPEEN**

(45) Date de publication et mention de la délivrance du brevet:
**14.04.2021 Bulletin 2021/15**

(51) Int Cl.:
***G01N 31/00*** *(2006.01)*     *G01N 33/20* *(2019.01)*

(21) Numéro de dépôt: **19192346.5**

(22) Date de dépôt: **19.08.2019**

(54) **PROCEDE DE DETERMINATION DE LA QUANTITE DE METAL ALCALIN OU ALCALINO-TERREUX CONTENU DANS UN MATERIAU**

VERFAHREN ZUR BESTIMMUNG DER ALKALI- ODER ERDALKALIMETALLMENGE IN EINEM MATERIAL

METHOD FOR DETERMINING THE AMOUNT OF ALKALI OR ALKALINE-EARTH METAL CONTAINED IN A MATERIAL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.08.2018 FR 1857601**

(43) Date de publication de la demande:
**26.02.2020 Bulletin 2020/09**

(73) Titulaire: **Commissariat à l'énergie atomique et aux énergies alternatives 75015 Paris (FR)**

(72) Inventeurs:
• **MAYOUSSE, Eric
  38054 GRENOBLE CEDEX 09 (FR)**
• **BLANC, Lionel
  38054 GRENOBLE CEDEX 09 (FR)**
• **CHAVILLON, Benoît
  38054 GRENOBLE CEDEX 09 (FR)**

(74) Mandataire: **Brevalex
  95, rue d'Amsterdam
  75378 Paris Cedex 8 (FR)**

(56) Documents cités:
**CN-U- 203 772 732     GB-A- 1 378 820**

• **BRACK P ET AL: "A Simple, Low-cost, and Robust System to Measure the Volume of Hydrogen Evolved by Chemical Reactions with Aqueous Solutions", JOURNAL OF VISUALIZED EXPERIMENTS, vol. 114, 17 août 2016 (2016-08-17), page E54383, XP055589569, DOI: 10.3791/54383**

EP 3 614 140 B1

**Description**

**DOMAINE TECHNIQUE**

La présente invention a trait à un procédé de détermination de la quantité de métal alcalin ou alcalino-terreux contenu dans un matériau *via* un procédé indirect dans lequel il est mesuré la quantité d'un produit issu de la réaction du métal alcalin ou alcalino-terreux avec un réactif approprié, la quantité mesurée étant ensuite corrélée avec la quantité de métal alcalin ou alcalino-terreux.

Ce procédé peut notamment trouver application dans les contextes suivants :

- la détermination de la quantité du métal alcalin ou alcalino-terreux, lorsque celui-ci se présente sous forme pure (c'est-à-dire sous forme non alliée avec un autre élément) ;
- l'estimation de la pureté d'un métal alcalin ou alcalino-terreux, en comparant la masse de métal alcalin ou alcalino-terreux déterminée par le procédé de l'invention et la masse pesée initialement ou en comparant la capacité théorique et la capacité déduite du procédé de l'invention ;
- la validation de la composition d'un alliage en métal alcalin ou alcalino-terreux en déterminant la quantité de métal alcalin ou alcalino-terreux contenue dans cet alliage par le procédé de l'invention ;
- la comparaison de l'état d'un matériau comprenant un métal alcalin ou alcalino-terreux à différents niveaux d'utilisation de ce matériau, ce contexte pouvant intervenir notamment dans le domaine des matériaux d'électrode comprenant un métal alcalin ou alcalino-terreux, dans lequel il peut être important d'estimer la quantité de métal alcalin ou alcalino-terreux impliquée dans la formation d'une couche de passivation ou de diagnostiquer un phénomène d'autodécharge en stockage ou en décharge.

Les métaux alcalins ou alcalino-terreux sont particulièrement réactifs, lorsqu'ils sont exposés à une atmosphère ambiante, notamment du fait de leur capacité à pouvoir réagir avec l'humidité de l'air, l'oxygène ou l'azote pour former des composés du type hydroxydes, oxydes ou nitrures. C'est notamment ce processus de réaction, qui intervient dans la formation d'une couche de passivation à la surface d'une électrode comprenant un métal alcalin ou alcalino-terreux au contact d'un électrolyte, la nature de la couche de passivation pouvant être différente en fonction du matériau d'électrode et du solvant d'électrolyte utilisé. Par exemple, en présence d'un électrolyte aqueux et d'une électrode en lithium, la couche de passivation formée comprend de l'hydroxyde de lithium LiOH, le lithium ainsi consommé pour la formation de la couche n'étant ainsi plus disponible comme matériau actif d'électrode pour le fonctionnement de la pile.

Pour vérifier l'état de fonctionnement d'une pile, il peut donc s'avérer important de pouvoir quantifier la quantité de matériau actif disponible, cette quantification pouvant s'effectuer, de manière indirecte, en quantifiant la couche de passivation formée de manière quantitative.

Pour ce faire, la quantité de couche de passivation formée peut être estimée, par exemple, en comparant l'électrode dans son état initial à l'électrode dans son état final, cette comparaison pouvant être effectuée selon différents modes de réalisation.

Selon un premier mode de réalisation, l'estimation de la quantité de couche de passivation peut être atteinte par mesure de la différence de masse entre l'électrode à l'état initial et l'électrode à l'état final. Toutefois, il n'est pas garanti que la valeur obtenue corresponde *stricto sensu* à la masse de la couche de passivation (ce qui permettrait de remonter à la quantité de matériau actif engagée dans la couche de passivation), la valeur obtenue pouvant comprendre, outre la masse de la couche de passivation, la masse d'électrolyte piégée dans cette couche de passivation. Les valeurs obtenues par ce mode de réalisation peuvent s'avérer donc faussées par rapport à la réalité de la situation et ainsi ne pas permettre d'accéder à la quantité réelle de matériau actif disponible pour le fonctionnement de la pile.

Selon un deuxième mode de réalisation, l'estimation de la quantité de couche de passivation peut être atteinte par mesure de la différence de masse entre l'électrode à l'état initial et l'électrode à l'état final mais en ayant préalablement procédé à l'élimination mécanique ou chimique de la couche de passivation avec toutefois les inconvénients suivants :

- la difficulté de s'assurer de l'élimination totale de la couche de passivation ;
- la difficulté de ne pas endommager le métal alcalin ou alcalino-terreux présent sous la couche de passivation ;
- la possibilité que le métal ainsi mis à nu par l'élimination de la couche de passivation réagisse de nouveau avec l'atmosphère environnante pour former une nouvelle couche de passivation.

La quantification indirecte du matériau actif disponible peut passer également par une caractérisation de la couche de passivation autrement que par sa quantité. Par exemple, il peut s'agir de déterminer l'épaisseur de la couche de passivation par des techniques microscopiques, telles que la microscopie électronique ou optique. Une fois l'épaisseur de la couche de passivation connue, il est possible d'accéder à l'épaisseur de la couche de matériau actif disponible donc à son volume. Ceci suppose toutefois que la couche de matériau actif soit homogène, ce qui n'est pas forcément garanti en fonction de la forme géométrique initiale du matériau et de ses conditions d'utilisation.

Face à la difficulté de mesurer la quantité de matériau actif disponible indirectement *via* la couche de passivation, il importe de pouvoir disposer de méthodes permettant de pouvoir quantifier la quantité de matériau actif du type métal alcalin ou alcalino-terreux par une technique impliquant une intervention directe sur ce matériau.

Des méthodes de dosage de métal alcalin ou alcalino-terreux ont déjà été proposés et notamment :

- une méthode de titration d'un métal alcalino-terreux, en particulier, le calcium, comme proposé dans Journal of the less-common Metals, 80, 1981, 241-255*, le calcium se présentant sous forme d'un alliage avec aluminium et étant mis à réagir avec de l'hydrogène, pour former un hydrure de calcium $CaH_2$, l'activité du calcium dans l'alliage étant ensuite obtenue à partir de données et relations thermodynamiques, cette méthode présentant toutefois pour inconvénient de devoir travailler sous pression d'hydrogène et à hautes températures ;
- une méthode de titration d'un métal alcalin, en particulier, le lithium, comme décrit dans Anal. Chem., 1968, 40 (11), p. 1731-1732*, où il s'agit de faire réagir du lithium avec du fluor et d'effectuer un dosage potentiométrique, cette méthode présentant toutefois des limitations du fait que des impuretés présentes dans le matériau à analyser peuvent réagir avec le fluor et fausser ainsi les résultats;
- une méthode de titration d'un métal alcalino-terreux, en particulier, le calcium, comme proposé dans le modèle d'utilité chinois CN 203 772 732 U qui décrit la détermination de la teneur en calcium d'un produit de tungstène, après la réaction de l'échantillon avec le peroxyde d'hydrogène et l'acide perchlorique pour la décomposition de l'échantillon; la quantité de calcium est déterminée par spectrométrie d'absorption atomique de la solution d'échantillon décomposée.

Au vu de ce qui existe déjà, les inventeurs se sont fixé pour objectif de mettre au point un procédé de détermination de la quantité de métal alcalin ou alcalino-terreux présent dans un matériau, qui soit de mise en œuvre en simple, ne nécessite pas d'appareillage complexe et de réactifs difficilement manipulables.

## EXPOSÉ DE L'INVENTION

Ainsi l'invention a trait à un procédé de détermination de la quantité de métal alcalin ou métal alcalino-terreux présent dans un matériau comprenant les étapes suivantes :

a) une étape de réaction quantitative du métal alcalin ou métal alcalino-terreux par mise en contact de celui-ci avec un milieu liquide comprenant un réactif porteur d'au moins une fonction -OH, moyennant quoi il se forme du dihydrogène et un hydroxyde ou oxyde de métal alcalin ou alcalino-terreux;

b) une étape de récolte de la quantité de dihydrogène émise lors de l'étape a) ;

c) une étape de détermination de la quantité de métal alcalin ou métal alcalino-terreux à partir de la quantité de dihydrogène récoltée lors de l'étape b).

Ainsi, le procédé comprend, en premier lieu, une étape de réaction quantitative du métal alcalin ou alcalino-terreux par mise en contact de celui-ci avec un milieu liquide comprenant un réactif porteur d'au moins une fonction -OH, moyennant quoi il se forme du dihydrogène et un hydroxyde ou oxyde de métal alcalin ou alcalino-terreux, la réaction préférentielle étant la réaction de formation d'un hydroxyde de métal alcalin ou alcalino-terreux.

Il s'entend classiquement que le métal alcalin ou alcalino-terreux dont la quantité est à déterminer selon le procédé de l'invention se présente au degré d'oxydation 0, ce qui n'exclut pas qu'il puisse être dans un matériau sous forme d'alliage avec un autre élément. En d'autres termes, le matériau comprenant le métal alcalin ou alcalino-terreux peut être constitué uniquement dudit métal alcalin ou alcalino-terreux ou peut être un alliage comprenant ledit métal alcalin ou alcalino-terreux et un ou plusieurs autres métaux.

Les métaux alcalins susceptibles d'être l'objet de la mise en œuvre du procédé de l'invention peuvent être du lithium, du sodium ou potassium, tandis que les métaux alcalino-terreux susceptibles d'être l'objet de la mise en œuvre du procédé de l'invention peuvent être du béryllium, du magnésium, du calcium, du strontium ou du baryum, ces métaux alcalins ou alcalino-terreux étant particulièrement abondants et énergétiques. Ces matériaux sont, de ce fait, particulièrement utilisés dans les domaines de l'énergie et notamment pour remplir la fonction de matériau actif dans des électrodes de batteries.

Le milieu liquide comprend (voire est constitué d') au moins un réactif porteur d'au moins une fonction -OH, ce réactif pouvant être en particulier de l'eau, un composé alcoolique ou des mélanges de ceux-ci.

Préférentiellement, le réactif est de l'eau, auquel cas l'étape de réaction conduit à un hydroxyde de métal alcalin ou alcalino-terreux. Dans le cas où le réactif est de l'eau, il peut être ajouté un acide pour faciliter la réaction avec le métal alcalin ou alcalino-terreux.

La réaction entre un métal alcalin ou alcalino-terreux (symbolisé par M ci-dessous) et de l'eau pour former un hydroxyde peut être symbolisée par l'équation chimique suivante :

$$M + H_2O \rightarrow \text{Hydroxyde de M} + aH_2$$

avec :

- M correspondant à un métal alcalin ou alcalino-terreux ;
- a correspondant au coefficient stœchiométrique affecté à $H_2$.

Avant la mise en œuvre de l'étape de réaction a), et pour affiner le résultat obtenu par le procédé de l'invention, le matériau comprenant le métal alcalin ou alcalino-terreux ou constitué uniquement de celui-ci peut être conditionné sous atmosphère inerte constituée d'un ou plusieurs gaz, par exemple, une atmosphère composée d'un ou plusieurs gaz inertes vis-à-vis du métal alcalin ou alcalino-terreux, ce ou ces gaz inertes pouvant être choisis parmi les gaz neutres (tels que l'argon, l'azote), l'air sec (c'est-à-dire dénué d'eau) et les mélanges de ceux-ci.

Plus spécifiquement, le matériau comprenant le métal alcalin ou alcalino-terreux ou constitué uniquement de celui-ci peut être conditionné dans un récipient clos comprenant une atmosphère inerte, par exemple, telle que définie ci-dessus, ce récipient clos étant ensuite ouvert pour la mise en œuvre de l'étape a) . La mise sous atmosphère inerte peut permettre, notamment, d'éviter une réaction du métal alcalin ou alcalino-terreux, dont on veut déterminer la quantité, avec l'air ambiant et de fausser ainsi la mesure, en particulier, s'il se forme au contact de l'air, une couche de passivation. Dans ce cas, après avoir été immergé dans le milieu liquide comprenant le réactif, ce récipient est ouvert de sorte à ce que le milieu liquide envahisse complètement son volume et soit en contact avec le matériau pour que la réaction puisse être engagée. Aussi, en d'autres termes, avant la mise en œuvre de l'étape a), le procédé de l'invention peut comprendre les étapes suivantes :

- une étape de conditionnement du matériau comprenant le métal alcalin ou alcalino-terreux sous atmosphère inerte dans un récipient clos ;
- une étape d'immersion du récipient clos dans le milieu liquide comprenant un réactif porteur d'au moins une fonction -OH ;
- une étape d'ouverture du récipient clos pour permettre la mise en contact du matériau avec le milieu liquide défini ci-dessus.

En variante de l'utilisation d'un récipient clos sous atmosphère inerte, avant la mise en œuvre de l'étape de réaction a), le matériau comprenant le métal alcalin ou alcalino-terreux ou constitué uniquement de celui-ci peut être conditionné dans un récipient clos sous vide et comprenant, éventuellement, en partie, une atmosphère inerte, lequel récipient clos est ensuite ouvert pour la mise en œuvre de l'étape a). Ceci nécessitera l'utilisation d'un récipient adapté pour le vide et pouvant comprendre, notamment, une vanne pour permettre de casser le vide et la mise en contact du matériau contenu dans le récipient avec le milieu liquide, pour que la réaction puisse s'effectuer. En d'autres termes, dans ce cas de figure, avant la mise en œuvre de l'étape a), le procédé de l'invention peut comprendre les étapes suivantes :

- une étape de conditionnement du matériau comprenant le métal alcalin ou alcalino-terreux dans un récipient clos sous vide et comprenant, éventuellement, en partie, une atmosphère inerte;
- une étape d'immersion du récipient dans le milieu liquide comprenant un réactif porteur d'au moins une fonction -OH ;
- une étape de rupture du vide pour permettre la mise en contact du matériau avec le milieu liquide défini ci-dessus.

Dans les cas précisés ci-dessus, à savoir, les cas où le matériau est conditionné sous atmosphère inerte, l'étape b) consistera, outre la récolte du dihydrogène issu de l'étape b), en la récolte du ou des gaz constitutif(s) de l'atmosphère inerte du récipient clos avant ouverture de celui-ci pour la mise en œuvre de l'étape a). Quant à l'étape c), elle consistera en une étape de détermination de la quantité de métal alcalin ou métal alcalino-terreux à partir de la quantité de gaz récoltés lors de l'étape b), ces gaz correspondant au dihydrogène formé lors de l'étape de réaction et au(x) gaz constitutif(s) de l'atmosphère inerte du récipient clos avant ouverture de celui-ci pour la mise en œuvre de l'étape a).

Une fois la réaction effectuée, il est procédé à la récolte de la quantité de dihydrogène émise lors de la réaction ; et à la récolte, le cas échéant, lorsque le matériau est conditionné initialement dans un récipient clos sous atmosphère inerte ou un récipient clos sous vide comprenant une atmosphère inerte, du ou des gaz constitutifs de l'atmosphère inerte du récipient clos comprenant le matériau comprenant le métal alcalin ou alcalino-terreux. Cette récolte peut être réalisée de différentes manières.

Plus spécifiquement, selon une première variante, l'étape de récolte du dihydrogène et, le cas échéant, du ou des gaz constitutif(s) de l'atmosphère inerte contenu(s) initialement dans le récipient clos susmentionné peut être effectuée au moyen d'une éprouvette graduée, la quantité de gaz récoltée étant mesurée en volume par lecture de la graduation imprimée sur l'éprouvette, le volume récolté correspondant ainsi au volume de dihydrogène produit lors de l'étape de réaction et, le cas échéant, au volume du ou des gaz constitutifs de l'atmosphère inerte du récipient clos suite à l'ouverture de celui-ci. Plus spécifiquement, lorsque le réactif est de l'eau, pour procéder à la collecte, l'éprouvette graduée peut être initialement complétement remplie d'eau puis retournée, extrémité ouverte vers le bas, sur le volume d'eau, dans lequel s'effectue la réaction avec le matériau comprenant le métal alcalin ou alcalino-terreux, l'éprouvette retournée étant placée, extrémité ouverte vers le bas, à la verticale de l'endroit occupé initialement par le matériau comprenant le métal alcalin ou alcalino-terreux. En d'autres termes, l'étape de récolte est réalisée *via* une éprouvette graduée initialement remplie d'eau, laquelle éprouvette est placée dans le milieu liquide, dans lequel s'effectue la réaction avec le métal alcalin ou alcalino-terreux contenu dans le matériau, extrémité ouverte vers le bas, à la verticale de l'endroit occupé par le matériau comprenant le métal alcalin ou alcalino-terreux. A l'issue de la réaction, le volume libre de la partie supérieure de l'éprouvette graduée correspond au volume de dihydrogène libéré par la réaction et, le cas échéant, au volume du ou des gaz constitutifs de l'atmosphère inerte du récipient clos suite à l'ouverture de celui-ci. Dans le cas où il y a utilisation d'un récipient clos sous vide, et lorsque le vide est très bon (par exemple, de l'ordre de 10 mbar), le récipient clos sous vide sera dénué d'atmosphère inerte et, de ce fait, le volume libre de la partie supérieure de l'éprouvette correspondra uniquement au volume total de dihydrogène libéré par la réaction.

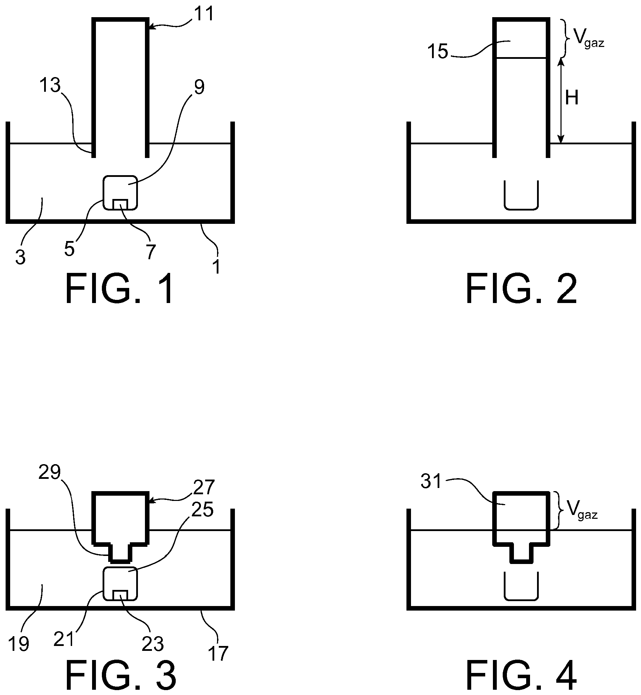

Les figures 1 et 2, jointes en annexe illustrent cette première variante avec un récipient clos sous atmosphère inerte avec pour la figure 1, la situation avant démarrage de la réaction et la figure 2, la situation à l'issue de la réaction.

La figure 1 représente spécifiquement :

- un réacteur 1 sous forme d'un récipient ouvert, dans lequel est placé un volume d'eau 3 pour la réaction avec le matériau comprenant le métal alcalin ou alcalino-terreux ;
- un récipient clos 5 renfermant le matériau 7 comprenant le métal alcalin ou alcalino-terreux et une atmosphère inerte 9, lequel récipient est immergé dans le volume d'eau du réacteur 1 ;
- une éprouvette 11 remplie d'eau et retournée, extrémité ouverte 13 vers le bas sur le volume d'eau du réacteur, l'extrémité ouverte 13 étant placée à la verticale du récipient clos 5.

La figure 2 représente les mêmes éléments que la figure 1, si ce n'est que :

- le récipient clos a été ouvert et le matériau a entièrement réagi avec l'eau ;
- l'éprouvette comprend une partie supérieure occupée par un volume de gaz 15 $V_{gaz}$ correspondant au volume de gaz de l'atmosphère inerte du récipient clos et au volume de dihydrogène produit par la réaction, la hauteur H correspondant à la hauteur mesurée entre le niveau d'eau affleurant du réacteur et le niveau d'eau dans l'éprouvette.

Cette définition de hauteur est également valable dans le cas où l'éprouvette est remplacée par un récipient de récolte tel que défini ci-dessus. Dans ce cas, la hauteur H correspond à la hauteur mesurée entre le niveau d'eau affleurant du réacteur et le niveau d'eau dans le récipient de récolte.

Selon une deuxième variante, lorsque le réactif est l'eau, l'étape de récolte du dihydrogène et, le cas échéant, du ou des gaz constitutif(s) de l'atmosphère inerte contenu(s) initialement dans le récipient clos peut être effectuée au moyen d'un récipient de récolte, la quantité de gaz ainsi récoltée pouvant ainsi être déterminée par différence de masse entre le récipient de récolte initialement complétement remplie d'eau avant récolte et le récipient dont une partie du volume d'eau initial est remplacée par le volume de gaz récolté, lequel correspond au volume total de dihydrogène formé lors de la réaction et, le cas échéant, au volume du ou des gaz constitutifs de l'atmosphère inerte du récipient clos suite à l'ouverture de celui-ci. Plus spécifiquement, pour procéder à la collecte des gaz, le récipient de récolte est initialement complétement rempli d'eau puis est retourné, extrémité ouverte vers le bas, sur le volume d'eau, dans lequel s'effectue la réaction avec le matériau comprenant le métal alcalin ou alcalino-terreux, le récipient retourné étant placé, extrémité ouverte vers le bas, à la verticale de l'endroit occupé initialement par le récipient comprenant le matériau comprenant le métal alcalin ou alcalino-terreux. En d'autres termes, l'étape de récolte est réalisée *via* un récipient de récolte initialement remplie d'eau, lequel récipient est disposé dans le milieu liquide, dans lequel s'effectue la réaction avec le métal alcalin ou alcalino-terreux contenu dans le matériau, extrémité ouverte vers le bas, à la verticale de l'endroit occupé par le matériau comprenant le métal alcalin ou alcalino-terreux. A l'issue de la réaction, le volume libre de la partie supérieure du récipient de récolte correspond au volume de dihydrogène libéré par la réaction et, et le cas échéant, au volume du ou des gaz constitutifs de l'atmosphère inerte du récipient clos après ouverture de celui-ci. Dans le cas où il y a utilisation d'un récipient clos sous vide, et lorsque le vide est très bon (par exemple, de l'ordre de 10 mbar), le récipient clos sous vide sera dénué d'atmosphère inerte et, de ce fait, le volume libre de la partie supérieure du récipient de récolte correspondra uniquement au volume total de dihydrogène libéré par la réaction.

Les figures 3 et 4, jointes en annexe illustrent cette deuxième variante avec un récipient clos sous atmosphère inerte avec pour la figure 3, la situation avant démarrage de la réaction et pour la figure 4, la situation à l'issue de la réaction.

La figure 3 représente spécifiquement :

- un réacteur 17 sous forme d'un récipient ouvert, dans lequel est placé un volume d'eau 19 pour la réaction avec le matériau comprenant le métal alcalin ou alcalino-terreux ;
- un récipient clos 21 renfermant le matériau 23 comprenant le métal alcalin ou alcalino-terreux et une atmosphère de gaz inerte 25, lequel récipient est immergé dans le volume d'eau du réacteur 19 ;
- un récipient à col 27 rempli d'eau jusqu'au col et retourné, extrémité ouverte 29 vers le bas sur le volume d'eau du réacteur, l'extrémité ouverte 29 étant placée à la verticale du récipient clos 21.

La figure 4 représente les mêmes éléments que la figure 1, si ce n'est que :

- le récipient clos a été ouvert et le matériau a entièrement réagi avec l'eau ;
- le récipient à col comprend une partie supérieure occupée par un volume de gaz 31 Vgaz correspondant au volume de gaz de l'atmosphère inerte du récipient clos et au volume de dihydrogène produit par la réaction.

Une fois le dihydrogène récolté, le procédé de l'invention comprend une étape de détermination de la quantité de métal alcalin ou alcalino-terreux à partir de la quantité de dihydrogène récoltée à l'étape b) et, le cas échéant, de gaz constitutif(s) de l'atmosphère inerte du récipient clos avant ouverture de celui-ci, et la quantité de métal alcalin ou métal alcalino-terreux.

Cette étape de détermination peut consister à établir une équation reliant la quantité de métal alcalin ou métal alcalino-terreux à la quantité de dihydrogène formé et, le cas échéant, de gaz constitutif(s) de l'atmosphère inerte du récipient clos avant ouverture de celui-ci et à appliquer cette équation pour obtenir cette quantité de métal alcalin ou alcalino-terreux, cette quantité correspondant avantageusement à la quantité molaire.

Ainsi, selon un premier mode de réalisation, dans le cas où :

- le matériau comprenant le métal alcalin ou alcalino-terreux est initialement conditionné, avant réaction, dans un récipient clos sous atmosphère inerte et le réactif est de l'eau ;
- le ou les gaz issus de l'atmosphère inerte du récipient clos et le dihydrogène produit sont récoltés dans une éprouvette graduée conformément aux modalités définies ci-dessus selon la première variante et illustrées sur les figures 1 et 2 ;

l'étape de détermination de la quantité de métal alcalin ou alcalino-terreux à partir de quantité récoltée de dihydrogène et du ou des gaz constitutifs de l'atmosphère inerte du récipient clos s'effectue *via* l'équation (I) suivante :

$$n_m = \frac{\dfrac{P_{gaz}}{T_{gaz}} \times V_{gaz} - \dfrac{P_{atm}}{T_{eau}} \times \dfrac{m_{eau}}{d_{eau}}}{a \times R - \dfrac{V_{mol\,m} \times P_{atm}}{T_{eau}}}$$

(I)

dans laquelle :

- $n_m$ correspond à la quantité de métal alcalin ou alcalino-terreux contenue dans le matériau, ladite quantité étant exprimée en moles ;
- $P_{gaz}$ correspond à la pression de gaz récolté dans l'éprouvette, ce gaz correspondant à un mélange du ou des gaz constitutifs de l'atmosphère inerte du récipient clos et de la totalité du dihydrogène produit lors de la réaction, la pression étant exprimée en mm de Hg ;
- $P_{atm}$ correspond à la pression atmosphérique, cette pression étant exprimée en mm de Hg ;
- $T_{gaz}$ correspond à la température du gaz récolté dans l'éprouvette, la température étant exprimée en K ;
- $V_{gaz}$ correspond au volume de gaz récolté dans l'éprouvette, ce volume correspondant à celui du mélange de gaz comprenant le ou les gaz constitutifs de l'atmosphère inerte du récipient clos et la totalité du dihydrogène produit lors de la réaction, le volume étant exprimé en cm³ ;
- $T_{eau}$ correspond à la température de l'eau contenue dans l'éprouvette, la température étant exprimée en K ;
- $m_{eau}$ correspond à la quantité d'eau déplacée correspondant à la différence ($m_B$-$m_A$), $m_B$ désignant la masse totale du récipient clos ayant tout son volume occupé par de l'eau (ce qui signifie, en d'autres termes, que le matériau a disparu totalement après réaction totale de celui-ci) et $m_A$ correspond à la masse totale du récipient clos comprenant le matériau et l'atmosphère inerte (c'est-à-dire avant ouverture du récipient et réaction du matériau), cette masse étant exprimée en gramme ;

- $d_{eau}$ correspond à la densité de l'eau ;
- a correspond au coefficient stœchiométrique affecté au dihydrogène dans l'équation de formation d'un hydroxyde du métal alcalin ou alcalino-terreux par l'eau sur la base d'une équation où le coefficient stœchiométrique affecté au métal est ramené à 1 ;
- R correspond à la constante des gaz parfaits (soit 62359 mm Hg.cm$^3$. °K$^{-1}$.mol$^{-1}$) ;
- $V_{mol\ m}$ correspond au volume molaire du métal alcalin ou alcalino-terreux (exprimé en cm$^3$/mol).

Plus spécifiquement, la pression $P_{gaz}$ peut être déterminée en connaissant la hauteur H dans l'éprouvette (telle que celle-ci est définie plus haut et désignée sur la figure 2) à l'issue de la réaction et en partant du principe que la pression d'un mm de mercure est la même que 13,6 mm d'eau, cette pression $P_{gaz}$ correspondant ainsi l'équation (II) suivante :

$$P_{gaz}=(P_{atm\text{-}}(H/13,6) - P_{eau})$$

$$(II)$$

avec $P_{eau}$ correspondant à la pression de l'eau contenue dans l'éprouvette, cette pression étant exprimée en mm de Hg.

L'équation (I) en y intégrant cette équation (II) peut être ainsi ramenée à l'équation (III) suivante :

$$n_m = \frac{\frac{V_{gaz}}{T_{gaz}} \times (P_{atm} - \frac{H}{13,6} - P_{eau}) - \frac{P_{atm}}{T_{eau}} \times \frac{m_{eau}}{d_{eau}}}{a \times R - \frac{V_{mol\ m} \times P_{atm}}{T_{eau}}}$$

$$(III)$$

A titre d'exemple, lorsque le métal dont on veut déterminer la quantité est le lithium, l'équation est ainsi l'équation (IV) suivante:

$$n_{Li} = \frac{\frac{V_{gaz}}{T_{gaz}} \times (P_{atm} - \frac{H}{13,6} - P_{eau}) - \frac{P_{atm}}{T_{eau}} \times \frac{m_{eau}}{d_{eau}}}{0,5 \times R - \frac{13 \times P_{atm}}{T_{eau}}}$$

$$(IV)$$

l'équation d'hydrolyse étant la suivante :

$$Li + H_2O \rightarrow Li(OH) + (1/2)H_2$$

et le volume molaire du lithium correspondant à 13 cm$^3$/mol.

La pression d'eau peut quant à elle être évaluée via la relation $P_{eau}=T_{eau}^2 * 0,0272638 + 6,664428$.

Selon un deuxième mode de réalisation, dans le cas où :

- le matériau comprenant le métal alcalin ou alcalino-terreux est initialement conditionné, avant réaction, dans un récipient clos sous vide comprenant, éventuellement, en partie une atmosphère inerte et le réactif est de l'eau ;
- le dihydrogène produit et, le cas échéant, le ou les gaz issus de l'atmosphère inerte du récipient clos, sont récoltés dans une éprouvette graduée conformément aux modalités définies ci-dessus selon la première variante et illustrées sur les figures 1 et 2 définies ci-dessus ;

l'étape de détermination de la quantité de métal alcalin ou alcalino-terreux à partir de la quantité de dihydrogène récolté et, le cas échéant, du ou des gaz issus de l'atmosphère inerte du récipient clos, s'effectue via l'équation (V) suivante :

$$n_m = \frac{\frac{V_{gaz}}{T_{gaz}} \times P gaz}{a \times R - \frac{V_{mol\,m} \times P_{atm}}{T_{eau}}}$$

(V)

dans laquelle :

- $n_m$ correspond à la quantité de métal alcalin ou alcalino-terreux contenue dans le matériau, ladite quantité étant exprimée en moles ;

- $P_{gaz}$ correspond à la pression de gaz récolté dans l'éprouvette, ce gaz correspondant, dans ce cas, à la totalité du dihydrogène produit lors de la réaction et, le cas échéant, au(x) gaz issu(s) de l'atmosphère inerte du récipient clos, la pression étant exprimée en mm de Hg ;

- $P_{atm}$ correspond à la pression atmosphérique, cette pression étant exprimée en mm de Hg ;

- $T_{gaz}$ correspond à la température du gaz récolté dans l'éprouvette, la température étant exprimée en K ;

- $V_{gaz}$ correspond au volume de gaz récolté dans l'éprouvette, ce volume correspondant à la totalité du dihydrogène produit lors de la réaction et, le cas échéant, du ou des gaz issus de l'atmosphère inerte du récipient clos, le volume étant exprimé en cm$^3$ ;

- a correspond au coefficient stœchiométrique affecté au dihydrogène dans l'équation de formation d'un hydroxyde de métal alcalin ou alcalino-terreux par l'eau sur la base d'une équation où le coefficient stœchiométrique affecté au métal est ramené à 1 ;

- R correspond à la constante des gaz parfaits (soit 62359 mm Hg.cm$^3$. °K$^{-1}$.mol$^{-1}$) ;

- $T_{eau}$ correspond à la température de l'eau contenue dans l'éprouvette, la température étant exprimée en K ;

- $V_{mol\,m}$ correspond au volume molaire du métal (exprimé en cm$^3$/mol).

Plus spécifiquement, la pression $P_{gaz}$ peut être déterminée en connaissant la hauteur H dans l'éprouvette (telle que celle-ci est définie plus haut et désignée sur la figure 2) à l'issue de la réaction et en partant du principe que la pression d'un mm de mercure est la même que 13,6 mm d'eau, cette pression $P_{gaz}$ correspondant ainsi l'équation (II) suivante :

$$P_{gaz} = (P_{atm} - (H/13,6) - P_{eau})$$

dans laquelle :

- H est tel que défini ci-dessus ;

- $P_{eau}$ correspond à la pression de l'eau contenue dans l'éprouvette, cette pression étant exprimée en mm de Hg.

L'équation (V) en y intégrant cette équation (II) peut être ainsi ramenée à l'équation (VI) suivante :

$$n_m = \frac{\frac{V_{gaz}}{T_{gaz}} \times (P_{atm} - \frac{H}{13,6} - P_{eau})}{a \times R - \frac{V_{mol\,m} \times P_{atm}}{T_{eau}}}$$

A titre d'exemple, lorsque le métal dont on veut déterminer la quantité est le lithium, l'équation est ainsi l'équation (VII) suivante :

$$n_{Li} = \frac{\dfrac{V_{gaz}}{T_{gaz}} \times (P_{atm} - \dfrac{H}{13,6} - P_{eau})}{0,5 \times R - \dfrac{13 \times P_{atm}}{T_{eau}}}$$

l'équation d'hydrolyse étant la suivante :

$$Li + H_2O \rightarrow Li(OH) + (1/2)H_2$$

et le volume molaire du lithium correspondant à 13 cm$^3$/mol.

La pression d'eau peut quant à elle être évaluée *via* la relation $P_{eau} = T_{eau}^2 * 0,0272638 + 6,664428$.

Selon un troisième mode de réalisation, dans le cas où :

- le matériau comprenant le métal alcalin ou alcalino-terreux est initialement conditionné, avant réaction, dans un récipient clos sous atmosphère inerte et le réactif est de l'eau ;
- le ou les gaz issus de l'atmosphère inerte du récipient clos et le dihydrogène produit sont récoltés dans un récipient de récolte conformément aux modalités définies ci-dessus selon la deuxième variante et illustrées sur les figures 3 et 4 mentionnées ci-dessus ;

l'étape de détermination de la quantité de métal alcalin ou alcalino-terreux à partir de la quantité de dihydrogène récolté et du ou des gaz issus de l'atmosphère inerte du récipient clos s'effectue *via* l'équation (VIII) suivante :

$$n_m = \frac{\dfrac{(mc - md)}{T_{gaz}} \times P_{gaz} - \dfrac{P_{atm}}{T_{eau}} \times \dfrac{m_{eau}}{d_{eau}}}{a \times R - \dfrac{V_{mol\,m} \times P_{atm}}{T_{eau}}}$$

(VIII)

dans laquelle :

- $n_m$ correspond à la quantité de métal alcalin ou alcalino-terreux contenue dans le matériau, ladite quantité étant exprimée en moles ;

- $P_{gaz}$ correspond à la pression de gaz récolté dans le récipient de récolte, ce gaz correspondant à un mélange du ou des gaz issus de l'atmosphère inerte du récipient clos et de la totalité du dihydrogène produit lors de la réaction, la pression étant exprimée en mm de Hg ;

- $P_{atm}$ correspond à la pression atmosphérique, cette pression étant exprimée en mm de Hg ;

- $T_{gaz}$ correspond à la température du gaz récolté dans le récipient de récolte, la température étant exprimée en K ;

- mc correspond à la masse du récipient de récolte rempli complètement d'eau ;

- md correspond à la masse du récipient de récolte après récolte du ou des gaz issus de l'atmosphère inerte du récipient clos et de la totalité du dihydrogène produit à l'issue de la réaction ;

- $T_{eau}$ correspond à la température de l'eau contenue dans le récipient de récolte, la température étant exprimée en K ;

- $m_{eau}$ correspond à la quantité d'eau déplacée correspondant à la différence ($m_B$-$m_A$), $m_B$ désignant la masse totale du récipient clos ayant tout son volume occupé par de l'eau (ce qui signifie, en d'autres termes, que le matériau a disparu totalement après réaction totale de celui-ci) et $m_A$ correspond à la masse totale du récipient clos comprenant le matériau et l'atmosphère inerte (c'est-à-dire avant ouverture du récipient et réaction du matériau), cette masse étant exprimée en gramme ;

- $d_{eau}$ correspond à la densité de l'eau ;

- a correspond au coefficient stœchiométrique affecté au dihydrogène dans l'équation de formation de l'hydroxyde de métal alcalin ou alcalino-terreux par l'eau sur la base d'une équation où le coefficient stœchiométrique affecté au métal est ramené à 1 ;

- R correspond à la constante des gaz parfaits (soit 62359 mm Hg.cm$^3$. $°K^{-1}$.mol$^{-1}$) ;

- $V_{mol\ m}$ correspond au volume molaire du métal (exprimé en cm$^3$/mol).

Plus spécifiquement, la pression $P_{gaz}$ peut être déterminée en connaissant la hauteur dans le récipient de récolte à l'issue de la réaction (à savoir, la hauteur mesurée entre le niveau d'eau affleurant du réacteur et le niveau d'eau dans le récipient de récolte) et en partant du principe que la pression d'un mm de mercure est la même que 13,6 mm d'eau, cette pression $P_{gaz}$ correspondant ainsi l'équation (II) suivante :

$$P_{gaz} = (P_{atm} - (H/13,6) - P_{eau})$$

dans laquelle :

- H est tel que défini ci-dessus ;

- $P_{eau}$ correspond à la pression de l'eau contenue dans le récipient de récolte, cette pression étant exprimée en mm de Hg.

L'équation (VIII) en y intégrant cette équation (II) peut être ainsi ramenée à l'équation (IX) suivante :

$$n_m = \frac{\frac{(mc - md)}{T_{gaz}} \times (P_{atm} - \frac{H}{13,6} - P_{eau}) - \frac{P_{atm}}{T_{eau}} \times \frac{m_{eau}}{d_{eau}}}{a \times R - \frac{V_{mol\ m} \times P_{atm}}{T_{eau}}}$$

$$(IX)$$

A titre d'exemple, lorsque le métal dont on veut déterminer la quantité est le lithium, l'équation est ainsi l'équation (X) suivante :

$$n_{Li} = \frac{\frac{mc - md}{T_{gaz}} \times (P_{atm} - \frac{H}{13,6} - P_{eau}) - \frac{P_{atm}}{T_{eau}} \times \frac{m_{eau}}{d_{eau}}}{0,5 \times R - \frac{13 \times P_{atm}}{T_{eau}}}$$

$$(X)$$

l'équation d'hydrolyse étant la suivante :

$$Li + H_2O \rightarrow Li(OH) + (1/2)H_2$$

et le volume molaire du lithium correspondant à 13 cm$^3$/mol.

La pression d'eau peut quant à elle être évaluée via la relation $P_{eau} = T_{eau}^2 * 0,0272638 + 6,664428$.

Selon un quatrième mode de réalisation, dans le cas où :

- le matériau comprenant le métal alcalin ou alcalino-terreux est initialement conditionné, avant réaction, dans un récipient clos sous vide comprenant, éventuellement, en partie une atmosphère inerte et le réactif est de l'eau ;
- le dihydrogène produit et, le cas échéant, le ou les gaz issus de l'atmosphère inerte du récipient clos est récolté dans un récipient de récolte conformément aux modalités définies ci-dessus selon la deuxième variante et illustrées sur les figures 3 et 4 définies ci-dessus ;

l'étape de détermination de la quantité de métal alcalin ou alcalino-terreux à partir de la quantité de dihydrogène récolté et, le cas échéant, du ou des gaz issus de l'atmosphère inerte du récipient clos, s'effectue *via* l'équation (XI) suivante :

$$n_m = \frac{\dfrac{(mc - md)}{T_{gaz}} \times P_{gaz}}{a \times R - \dfrac{V_{mol\,m} \times P_{atm}}{T_{eau}}}$$

(XI)

dans laquelle :

- $n_m$ correspond à la quantité de métal alcalin ou alcalino-terreux contenue dans le matériau, ladite quantité étant exprimée en moles ;

- $P_{gaz}$ correspond à la pression de gaz récolté dans le récipient de récolte, ce gaz correspondant à la totalité du dihydrogène produit lors de la réaction et, le cas échéant, au(x) gaz issu(s) de l'atmosphère inerte du récipient clos, la pression étant exprimée en mm de Hg ;

- $P_{atm}$ correspond à la pression atmosphérique, cette pression étant exprimée en mm de Hg ;

- $T_{gaz}$ correspond à la température du gaz récolté dans le récipient de récolte, la température étant exprimée en K ;

- mc correspond à la masse du récipient de récolte rempli complètement d'eau ;

- md correspond à la masse du récipient de récolte après récolte du gaz inerte et de la totalité du dihydrogène produit à l'issue de la réaction ;

- $T_{eau}$ correspond à la température de l'eau contenue dans le récipient de récolte, la température étant exprimée en K ;

- a correspond au coefficient stœchiométrique affecté au dihydrogène dans l'équation de formation d'hydroxyde du métal alcalin ou alcalino-terreux par l'eau sur la base d'une équation où le coefficient stœchiométrique affecté au métal est ramené à 1 ;

- R correspond à la constante des gaz parfaits (soit 62359 mm Hg.cm$^3$. °K$^{-1}$.mol$^{-1}$) ;

- $V_{mol\,m}$ correspond au volume molaire du métal (exprimé en cm$^3$/mol).

Plus spécifiquement, lorsque le niveau de gaz récolté dans le récipient clos correspond au niveau du volume d'eau du réacteur (soit, en d'autre termes, lorsque H est égal à 0 comme illustré sur la figure 4) la pression $P_{gaz}$ correspond à $P_{atm}$-$P_{eau}$ avec $P_{eau}$ correspondant à la pression de l'eau contenue dans l'éprouvette, cette pression étant exprimée en mm de Hg.

L'équation (XI) peut être ainsi ramenée à l'équation (XII) suivante :

$$n_m = \frac{\dfrac{(mc - md)}{T_{gaz}} \times (P_{atm} - P_{eau})}{a \times R - \dfrac{V_{mol\,m} \times P_{atm}}{T_{eau}}}$$

(XII)

dans laquelle $P_{eau}$ correspond à la pression de l'eau contenue dans le récipient de récolte, cette pression étant exprimée en mm de Hg.

A titre d'exemple, lorsque le métal dont on veut déterminer la quantité est le lithium, l'équation est ainsi l'équation (XIII) suivante :

$$n_{Li} = \frac{\dfrac{mc - md}{T_{gaz}} \times (P_{atm} - P_{eau})}{0{,}5 \times R - \dfrac{13 \times P_{atm}}{T_{eau}}}$$

(XIII)

l'équation d'hydrolyse étant la suivante :

$$Li + H_2O \rightarrow Li(OH) + (1/2)H_2$$

et le volume molaire du lithium correspondant à 13 cm$^3$/mol.

La pression d'eau peut quant à elle être évaluée via la relation $P_{eau} = T_{eau}^2 * 0{,}0272638 + 6{,}664428$.

Le troisième mode de réalisation et le quatrième mode de réalisation nécessitant une pesée pour la détermination des valeurs de mc et md nécessite l'utilisation d'une balance et peut permettre une plus grande précision que le premier mode et deuxième mode nécessitant une lecture visuelle du volume de gaz dégagé. Ces troisième et quatrième modes de réalisation peuvent donc s'avérer particulièrement avantageux lorsqu'il s'agit de déterminer de faibles quantités de métal alcalin ou alcalino-terreux.

A partir de la quantité de métal alcalin ou alcalino-terreux en termes de nombre de moles, il est possible d'en déduire *via* l'équation d'hydrolyse le nombre de moles d'électrons échangés au cours de la réaction et, à partir de ce nombre de moles d'électrons, la capacité du métal alcalin ou alcalino-terreux, exprimée en Ah (c'est-à-dire en ampères-heures).

Ainsi, l'invention a trait à un procédé de détermination de la capacité d'un métal alcalin ou alcalino-terreux contenu dans un matériau comprenant les étapes suivantes :

d) une étape de mise en œuvre du procédé de détermination de la quantité molaire d'un métal alcalin ou alcalino-terreux contenu dans un matériau, le procédé étant tel que défini ci-dessus ;

e) à partir du la quantité obtenue en d), une étape de détermination du nombre de moles d'électrons échangés lors de l'étape de réaction a) du procédé de détermination de la quantité molaire ;

f) à partir du nombre de moles d'électrons obtenu en e), une étape de détermination de la capacité en ampères-heures.

Ce procédé est particulièrement adapté pour les métaux alcalins ou alcalino-terreux lorsqu'ils sont utilisés dans les domaines liés à la production d'énergie, notamment lorsqu'ils sont utilisés comme matériaux actifs pour batteries.

L'étape e) de détermination du nombre d'électrons échangés lors de l'étape a) à partir de la quantité molaire obtenue en d) peut être réalisée en se basant sur l'équation de formation de l'hydroxyde du métal alcalin ou alcalino-terreux, dans laquelle le métal est affecté d'un coefficient stœchiométrique égal à 1.

Ainsi, à titre d'exemple, pour les métaux alcalins donnant des cations monovalents, comme Li, l'équation de formation de l'hydroxyde du métal alcalin en présence d'eau est la suivante :

$$Li + H_2O \rightarrow LiOH + (1/2)H_2$$

ce qui correspond à la somme des deux demi-équations suivantes :

$$Li \rightarrow Li^+ + e^- \text{ et } H_2O + e^- \rightarrow OH^- + (1/2)H_2$$

le nombre de moles d'électrons échangés correspondant au nombre de moles de lithium (soit la quantité molaire déterminée à l'étape d) mentionnée ci-dessus).

Quant aux métaux alcalino-terreux donnant des cations bivalents, comme Ca, l'équation de formation de l'hydroxyde du métal alcalino-terreux en présence d'eau est la suivante :

$$Ca + 2H_2O \rightarrow Ca(OH)_2 + H_2$$

ce qui correspond à la somme des deux demi-équations suivantes :

$$Ca \rightarrow Ca^{2+} + 2e^- \text{ et } 2H_2O + 2e^- \rightarrow 2OH^- + H_2$$

le nombre de moles d'électrons échangés correspondant à 2 fois le nombre de moles de calcium (soit la quantité molaire déterminée à l'étape d) mentionnée ci-dessus).

Enfin, une fois le nombre de moles d'électrons échangé connu, la capacité en ampères-heures peut être facilement déterminée en partant du principe que 1 mole d'électrons échangé correspond à 26,8 Ampères-heures.

L'invention va maintenant être décrite par rapport aux exemples suivants donnés à titre illustratif et non limitatif.

## BRÈVE DESCRIPTION DES DESSINS

Les figures 1 et 2, jointes en annexe illustrent la première variante mentionnée ci-dessus avec pour la figure 1, la situation avant démarrage de la réaction et la figure 2, la situation à l'issue de la réaction.

Les figures 3 et 4, jointes en annexe illustrent la troisième variante mentionnée ci-dessus avec pour la figure 1, la situation avant démarrage de la réaction et la figure 2, la situation à l'issue de la réaction.

## EXPOSÉ DÉTAILLÉ D'UN MODE DE REALISATION PARTICULIER

### EXEMPLE 1

Cet exemple a pour visée de prouver la fiabilité du procédé de l'invention pour vérifier la pureté d'un échantillon de lithium de 51 mg en comparant la capacité théorique à la capacité obtenue par la mise en œuvre du procédé de l'invention.

Pour ce faire, l'échantillon de lithium de 51 mg est initialement placé dans un récipient clos sous atmosphère inerte puis est ensuite placé dans un réacteur comprenant de l'eau. Le récipient clos est ouvert pour permettre l'eau d'entrer en contact avec le matériau pour former l'hydroxyde de lithium Li(OH). Le dihydrogène est récupéré dans un récipient de récolte à col et la quantité de dihydrogène et par la même occasion de métal lithium est déterminée conformément aux modalités du troisième mode de réalisation exposé ci-dessus.

L'équation de détermination à appliquer est la suivante :

$$n_{Li} = \frac{\dfrac{(mc - md)}{T_{gaz}} \times \left(P_{atm} - \dfrac{H}{13,6} - P_{eau}\right) - \dfrac{P_{atm}}{T_{eau}} \times \dfrac{m_{eau}}{d_{eau}}}{a \times R - \dfrac{V_{mol\,m} \times P_{atm}}{T_{eau}}}$$

Dans ce cas de figure, H est égal à 0, a est égal à 0,5 et le nombre de moles de lithium correspond au nombre de moles d'électrons.

L'équation pour obtenir le nombre de moles d'électrons est donc la suivante :

$$n_{e-} = \frac{\frac{(m_c - m_{D)}}{T_{gaz}} \times (P_{atm} - P_{eau}) - \frac{P_{atm}}{T_{eau}} \times m_{eau}}{0,5 \times R - \frac{13 \times P_{atm}}{T_{eau}}}$$

Le récipient à col rempli complètement d'eau présente une masse $m_C$ = 221 mg

Le récipient à col après réaction contenant le gaz présente une masse $m_D$ = 121 mg

Le volume de gaz est déterminé à partir de me - $m_D$ et de la densité de l'eau de 1, ainsi $V_{gaz}$ = 100 cm$^3$.

Les mesures de $T_{eau}$ = 289 K, de $T_{gaz}$ = 293 K, de la pression atmosphérique $P_{atm}$ (mmHg) (766 mm Hg) et la pression de l'eau $P_{eau}$ (17,55 mm Hg) permettent de déterminer le nombre de mole d'électrons, qui correspond à 7,342 mmol.

Le nombre de mole d'électrons permet de déterminer le nombre de mAh quantifiés, soit ici 196,7 mAh ce qui est très proche de la valeur théorique.

Cet exemple permet de conclure que l'échantillon de lithium était très pur.


EXEMPLE 2

Cet exemple a pour visée de prouver la fiabilité du procédé de l'invention pour estimer l'état d'une électrode de calcium après avoir été utilisée dans une pile.

Plus spécifiquement, l'électrode de calcium présente initialement une masse de 3,66 g et une capacité de 4,897 Ah (ou 1,338 Ah/g). L'électrode de calcium est ensuite placée dans un électrolyte au chlorure de thionyle dans une pile primaire de format C.

La pile est déchargée sous 50 mA à 150°C jusqu'à 0,6 V. La décharge dure 38,5 heures, soit une capacité déchargée de 1924 mAh.

La pile est alors démontée, le chlorure de thionyle est éliminé par nettoyage avec du dichlorométhane. L'électrode de calcium ainsi récupérée est mise dans un récipient qui se ferme de façon étanche sous air anhydre. Visuellement, l'électrode de calcium semble comporter en surface une importante couche de passivation.

Le récipient comprenant l'électrode est ensuite immergé dans un réacteur comprenant de l'eau puis il est ouvert pour permettre la mise en contact de l'électrode avec l'eau et le dihydrogène produit est récolté dans une éprouvette graduée selon les modalités définies dans le premier mode de réalisation ci-dessus.

La détermination de la quantité de calcium contenue dans l'électrode ainsi utilisée est alors effectuée *via* l'équation suivante :

$$n_{Ca} = \frac{\frac{V_{gaz}}{T_{gaz}} \times (P_{atm} - \frac{H}{13,6} - P_{eau}) - \frac{P_{atm}}{T_{eau}} \times \frac{m_{eau}}{d_{eau}}}{R - \frac{26 \times P_{atm}}{T_{eau}}}$$

Vgaz=790 cm$^3$; Tgaz=292 K ; Patm=765 mm Hg ; H=80 mm ; $T_{eau}$=287 K ; $m_{eau}$=64,4 g soit 29,5 mmol de calcium.

La quantité de moles de calcium étant déterminée, l'on en déduit le nombre de moles d'électrons échangés lors de la réaction $n_{e-}$, qui correspond à 2*$n_{Ca}$, ce qui permet de remonter la capacité restante de 1582 mAh.

La capacité initiale étant de 4897 mAh et la décharge de la pile étant de 1924 mAh, la capacité restante devrait être de 2973 mAh. Or elle n'est évaluée qu'à 1582 mAh. Le différentiel de 1391 mAh peut être attribué au phénomène d'autodécharge directe du calcium avec l'électrolyte, ce qui permet d'expliquer l'importante couche de passivation présente à la surface de l'électrode.

Ainsi, le procédé de l'invention peut être mis à profit pour déterminer des autodécharges en décharge ou en stockage pour différentes technologies utilisant un métal alcalin ou alcalino-terreux.

Le procédé de l'invention pourrait également être mis à profit pour permettre d'étudier la réactivité d'un métal alcalin ou alcalino-terreux dans un nouvel électrolyte, en vue, par exemple, d'en améliorer la stabilité.


**Revendications**

1. Procédé de détermination de la quantité de métal alcalin ou métal alcalino-terreux présent dans un matériau (7 ; 23) comprenant les étapes suivantes :

a) une étape de réaction quantitative du métal alcalin ou métal alcalino-terreux par mise en contact de celui-ci avec un milieu liquide comprenant un réactif porteur d'au moins une fonction -OH, moyennant quoi il se forme du dihydrogène et un hydroxyde ou oxyde de métal alcalin ou alcalino-terreux;

b) une étape de récolte de la quantité de dihydrogène émise lors de l'étape a) ;

c) une étape de détermination de la quantité de métal alcalin ou métal alcalino-terreux à partir de la quantité de dihydrogène récoltée lors de l'étape b).

2. Procédé selon la revendication 1, dans lequel le réactif porteur d'au moins une fonction -OH est de l'eau (3; 19), un composé alcoolique et des mélanges de ceux-ci.

3. Procédé selon la revendication 1 ou 2, dans lequel, avant la mise en œuvre de l'étape a), le matériau comprenant le métal alcalin ou alcalino-terreux est conditionné dans un récipient clos (5; 21) comprenant une atmosphère inerte (9; 25), lequel récipient clos est ensuite ouvert pour la mise en œuvre de l'étape a).

4. Procédé selon l'une quelconque des revendications 1 à 2, dans lequel, avant la mise en œuvre de l'étape a), le matériau comprenant le métal alcalin ou alcalino-terreux est conditionné dans un récipient clos sous vide et comprenant, éventuellement, en partie, une atmosphère inerte, lequel récipient clos est ensuite ouvert pour la mise en œuvre de l'étape a).

5. Procédé selon la revendication 3 ou 4, dans lequel l'étape b) consiste, outre la récolte du dihydrogène issu de l'étape b), en la récolte du ou des gaz constitutif(s) de l'atmosphère inerte du récipient clos avant ouverture de celui-ci pour la mise en œuvre de l'étape a).

6. Procédé selon la revendication 3 ou 4, dans lequel l'étape c) consiste en une étape de détermination de la quantité de métal alcalin ou métal alcalino-terreux à partir de la quantité de gaz récoltés lors de l'étape b) et, ces gaz correspondant au dihydrogène formé lors de l'étape de réaction et au(x) gaz constitutif(s) de l'atmosphère inerte du récipient clos avant ouverture de celui-ci pour la mise en œuvre de l'étape a).

7. Procédé selon l'une quelconque des revendications précédentes, dans lequel l'étape de récolte du dihydrogène et, le cas échéant, du ou des gaz constitutif(s) de l'atmosphère inerte contenu(s) initialement dans le récipient clos est effectuée au moyen d'une éprouvette graduée (11), la quantité de gaz ainsi récolté étant mesurée en volume par lecture de la graduation imprimée sur l'éprouvette.

8. Procédé selon la revendication 7, dans lequel, lorsque le réactif porteur d'au moins une fonction -OH est de l'eau (3; 19), l'étape de récolte est réalisée *via* une éprouvette graduée initialement complètement remplie d'eau, laquelle éprouvette est placée dans le milieu liquide, dans lequel s'effectue la réaction avec le métal alcalin ou alcalino-terreux contenu dans le matériau, extrémité ouverte (13) vers le bas, à la verticale de l'endroit occupé par le matériau comprenant le métal alcalin ou alcalino-terreux.

9. Procédé selon l'une quelconque des revendications 1 à 6, dans lequel l'étape de récolte est réalisé au moyen d'un récipient de récolte, la quantité de gaz ainsi récoltée étant déterminée par différence de masse entre le récipient de récolte initialement complètement remplie d'eau avant récolte et le récipient dont une partie du volume d'eau contenu initialement dans le récipient est remplacée par le volume de gaz récolté (15; 31).

10. Procédé selon la revendication 9, dans lequel, lorsque le réactif porteur d'au moins une fonction -OH est de l'eau, l'étape de récolte est réalisée *via* un récipient de récolte initialement remplie d'eau, lequel récipient est disposé dans le milieu liquide, dans lequel s'effectue la réaction avec le métal alcalin ou alcalino-terreux contenu dans le matériau, extrémité ouverte vers le bas, à la verticale de l'endroit occupé par le matériau comprenant le métal alcalin ou alcalino-terreux.

11. Procédé selon la revendication 3, dans lequel, lorsque l'étape de récolte est telle que définie à la revendication 8, l'étape de détermination de la quantité de métal alcalin ou alcalino-terreux à partir de la quantité récoltée de dihydrogène et du ou des gaz constitutifs de l'atmosphère inerte du récipient clos s'effectue *via* l'équation (I) suivante :

$$n_m = \frac{\dfrac{P_{gaz}}{T_{gaz}} \times V_{gaz} - \dfrac{P_{atm}}{T_{eau}} \times \dfrac{m_{eau}}{d_{eau}}}{a \times R - \dfrac{V_{mol\,m} \times P_{atm}}{T_{eau}}}$$

(I)

dans laquelle :

- $n_m$ correspond à la quantité de métal alcalin ou alcalino-terreux contenue dans le matériau, ladite quantité étant exprimée en moles ;
- $P_{gaz}$ correspond à la pression de gaz récolté dans l'éprouvette, ce gaz correspondant à un mélange du ou des gaz constitutifs de l'atmosphère inerte du récipient clos et de la totalité du dihydrogène produit lors de la réaction, la pression étant exprimée en mm de Hg ;
- $P_{atm}$ correspond à la pression atmosphérique, cette pression étant exprimée en mm de Hg ;
- $T_{gaz}$ correspond à la température du gaz récolté dans l'éprouvette, la température étant exprimée en K ;
- $V_{gaz}$ correspond au volume de gaz récolté dans l'éprouvette, ce volume correspondant à celui du mélange de gaz comprenant le ou les gaz constitutifs de l'atmosphère inerte du récipient clos et la totalité du dihydrogène produit lors de la réaction, le volume étant exprimé en $cm^3$ ;
- $T_{eau}$ correspond à la température de l'eau contenue dans l'éprouvette, la température étant exprimée en K ;
- $m_{eau}$ correspond à la quantité d'eau déplacée correspondant à la différence ($m_B$-$m_A$), $m_B$ désignant la masse totale du récipient clos ayant tout son volume occupé par de l'eau et $m_A$ correspond à la masse totale du récipient clos comprenant le matériau et l'atmosphère inerte, cette masse étant exprimée en gramme ;
- $d_{eau}$ correspond à la densité de l'eau ;
- $a$ correspond au coefficient stœchiométrique affecté au dihydrogène dans l'équation de formation d'un hydroxyde du métal alcalin ou alcalino-terreux par l'eau sur la base d'une équation où le coefficient stœchiométrique affecté au métal est ramené à 1 ;
- R correspond à la constante des gaz parfaits (soit 62359 mm $Hg.cm^3$. $°K^{-1}.mol^{-1}$) ;
- $V_{mol\,m}$ correspond au volume molaire du métal alcalin ou alcalino-terreux (exprimé en $cm^3/mol$).

12. Procédé selon la revendication 4, dans lequel, lorsque l'étape de récolte est telle que définie à la revendication 8, l'étape de détermination de la quantité de métal alcalin ou alcalino-terreux à partir de la quantité de dihydrogène récolté et, le cas échéant, du ou des gaz issus de l'atmosphère inerte du récipient clos, s'effectue *via* l'équation (V) suivante :

$$n_m = \frac{\dfrac{V_{gaz}}{T_{gaz}} \times P{gaz}}{a \times R - \dfrac{V_{mol\,m} \times P_{atm}}{T_{eau}}}$$

(V)

dans laquelle :

- $n_m$ correspond à la quantité de métal alcalin ou alcalino-terreux contenue dans le matériau, ladite quantité étant exprimée en moles ;
- $P_{gaz}$ correspond à la pression de gaz récolté dans l'éprouvette, ce gaz correspondant, dans ce cas, à la totalité du dihydrogène produit lors de la réaction et, le cas échéant, au(x) gaz issu(s) de l'atmosphère inerte du récipient clos, la pression étant exprimée en mm de Hg ;
- $P_{atm}$ correspond à la pression atmosphérique, cette pression étant exprimée en mm de Hg ;
- $T_{gaz}$ correspond à la température du gaz récolté dans l'éprouvette, la température étant exprimée en K ;
- $V_{gaz}$ correspond au volume de gaz récolté dans l'éprouvette, ce volume correspondant à la totalité du dihydrogène produit lors de la réaction et, le cas échéant, du ou des gaz issus de l'atmosphère inerte du récipient clos, le volume étant exprimé en $cm^3$ ;
- $a$ correspond au coefficient stœchiométrique affecté au dihydrogène dans l'équation de formation d'un hydroxyde de métal alcalin ou alcalino-terreux par l'eau sur la base d'une équation où le coefficient stœchiométrique affecté au métal est ramené à 1 ;
- R correspond à la constante des gaz parfaits (soit 62359 mm Hg.cm$^3$. °K$^{-1}$.mol$^{-1}$) ;
- T$_{eau}$ correspond à la température de l'eau contenue dans l'éprouvette, la température étant exprimée en K ;
- V$_{mol\ m}$ correspond au volume molaire du métal (exprimé en cm$^3$/mol).

13. Procédé selon la revendication 3, dans lequel, lorsque l'étape de récolte est telle que définie à la revendication 10, dans lequel l'étape de détermination de la quantité de métal alcalin ou alcalino-terreux à partir de la quantité de dihydrogène récolté et du ou des gaz issus de l'atmosphère inerte du récipient clos s'effectue *via* l'équation (VIII) suivante :

$$n_m = \frac{\frac{(mc-md)}{T_{gaz}} \times P_{gaz} - \frac{P_{atm}}{T_{eau}} \times \frac{m_{eau}}{d_{eau}}}{a \times R - \frac{V_{mol\ m} \times P_{atm}}{T_{eau}}}$$

(VIII)

dans laquelle :

- n$_m$ correspond à la quantité de métal alcalin ou alcalino-terreux contenue dans le matériau, ladite quantité étant exprimée en moles ;
- P$_{gaz}$ correspond à la pression de gaz récolté dans le récipient de récolte, ce gaz correspondant à un mélange du ou des gaz issus de l'atmosphère inerte du récipient clos et de la totalité du dihydrogène produit lors de la réaction, la pression étant exprimée en mm de Hg ;
- P$_{atm}$ correspond à la pression atmosphérique, cette pression étant exprimée en mm de Hg ;
- T$_{gaz}$ correspond à la température du gaz récolté dans le récipient de récolte, la température étant exprimée en K ;
- mc correspond à la masse du récipient de récolte rempli complètement d'eau ;
- md correspond à la masse du récipient de récolte après récolte du ou des gaz issus de l'atmosphère inerte du récipient clos et de la totalité du dihydrogène produit à l'issue de la réaction ;
- T$_{eau}$ correspond à la température de l'eau contenue dans le récipient de récolte, la température étant exprimée en K ;
- m$_{eau}$ correspond à la quantité d'eau déplacée correspondant à la différence (m$_B$-m$_A$), m$_B$ désignant la masse totale du récipient clos ayant tout son volume occupé par de l'eau et m$_A$ correspond à la masse totale du récipient clos comprenant le matériau et l'atmosphère inerte ;
- d$_{eau}$ correspond à la densité de l'eau ;
- a correspond au coefficient stœchiométrique affecté au dihydrogène dans l'équation de formation de l'hydroxyde de métal alcalin ou alcalino-terreux par l'eau sur la base d'une équation où le coefficient stœchiométrique affecté au métal est ramené à 1 ;
- R correspond à la constante des gaz parfaits (soit 62359 mm Hg.cm$^3$. °K$^{-1}$.mol$^{-1}$) ;
- V$_{mol\ m}$ correspond au volume molaire du métal (exprimé en cm$^3$/mol).

14. Procédé selon la revendication 4, dans lequel, lorsque l'étape de récolte est telle que définie à la revendication 10, l'étape de détermination de la quantité de métal alcalin ou alcalino-terreux à partir de la quantité de dihydrogène récolté et, le cas échéant, du ou des gaz issus de l'atmosphère inerte du récipient clos, s'effectue *via* l'équation (XI) suivante :

$$n_m = \frac{\frac{(mc-md)}{T_{gaz}} \times P_{gaz}}{a \times R - \frac{V_{mol\ m} \times P_{atm}}{T_{eau}}}$$

(XI)

dans laquelle :

- $n_m$ correspond à la quantité de métal alcalin ou alcalino-terreux contenue dans le matériau, ladite quantité étant exprimée en moles ;
- $P_{gaz}$ correspond à la pression de gaz récolté dans le récipient de récolte, ce gaz correspondant à la totalité du dihydrogène produit lors de la réaction et, le cas échéant, au(x) gaz issu(s) de l'atmosphère inerte du récipient clos, la pression étant exprimée en mm de Hg ;
- $P_{atm}$ correspond à la pression atmosphérique, cette pression étant exprimée en mm de Hg ;
- $T_{gaz}$ correspond à la température du gaz récolté dans le récipient de récolte, la température étant exprimée en K ;
- mc correspond à la masse du récipient de récolte rempli complètement d'eau ;
- md correspond à la masse du récipient de récolte après récolte du gaz inerte et de la totalité du dihydrogène produit à l'issue de la réaction ;
- $T_{eau}$ correspond à la température de l'eau contenue dans le récipient de récolte, la température étant exprimée en K ;
- a correspond au coefficient stœchiométrique affecté au dihydrogène dans l'équation de formation d'hydroxyde du métal alcalin ou alcalino-terreux par l'eau sur la base d'une équation où le coefficient stœchiométrique affecté au métal est ramené à 1 ;
- R correspond à la constante des gaz parfaits (soit 62359 mm Hg.cm$^3$. °K$^{-1}$.mol$^{-1}$) ;
- $V_{mol\ m}$ correspond au volume molaire du métal (exprimé en cm$^3$/mol).

15. Procédé de détermination de la capacité d'un métal alcalin ou alcalino-terreux contenu dans un matériau comprenant les étapes suivantes :

d) une étape de mise en œuvre du procédé de détermination de la quantité molaire d'un métal alcalin ou alcalino-terreux contenu dans un matériau, le procédé étant tel que défini selon l'une quelconque des revendications 1 à 14 ;
e) à partir du la quantité obtenue en d), une étape de détermination du nombre de moles d'électrons échangés lors de l'étape a) du procédé de détermination de la quantité molaire ;
f) à partir du nombre de moles d'électrons obtenu en e), une étape de détermination de la capacité en ampères-heures.

**Patentansprüche**

1. Verfahren zur Bestimmung der Menge an Alkalimetall oder Erdalkalimetall, die in einem Material (7; 23) vorhanden ist, umfassend die folgenden Schritte:

a) einen Schritt der quantitativen Reaktion des Alkalimetalls oder Erdalkalimetalls durch Inkontaktbringen mit einem flüssigen Medium, das ein Reagenz enthält, das mindestens eine -OH-Funktion trägt, wodurch Dihydrogen und ein Alkalimetall- oder Erdalkalimetallhydroxid oder -oxid gebildet werden;
b) einen Schritt des Sammelns der in Schritt a) emittierten Menge an Dihydrogen;
c) einen Schritt der Bestimmung der Menge an Alkali- oder Erdalkalimetall aus der in Schritt b) gesammelten Menge an Dihydrogen.

2. Verfahren nach Anspruch 1, wobei das Reagenz, das mindestens eine -OH-Funktion trägt, Wasser (3; 19), eine alkoholische Verbindung und Mischungen davon ist.

3. Verfahren nach Anspruch 1 oder 2, wobei das das Alkali- oder Erdalkalimetall enthaltende Material vor der Ausführung von Schritt a) in einem geschlossenen Behälter (5; 21) mit inerter Atmosphäre (9; 25) aufbewahrt wird, welcher geschlossene Behälter dann zur Ausführung von Schritt a) geöffnet wird.

4. Verfahren nach einem der Ansprüche 1 bis 2, wobei vor der Ausführung von Schritt a) das das Alkali- oder Erdalkalimetall enthaltende Material in einem geschlossenen Behälter unter Vakuum aufbewahrt wird, der gegebenenfalls teilweise eine inerte Atmosphäre enthält, und der geschlossene Behälter dann zur Ausführung von Schritt a) geöffnet wird.

5. Verfahren nach Anspruch 3 oder 4, wobei Schritt b) zusätzlich zum Sammeln von Dihydrogen aus Schritt b) darin besteht, das Gas bzw. die Gase, die die inerte Atmosphäre des geschlossenen Behälters bilden, zu sammeln, bevor dieser zur Ausführung von Schritt a) geöffnet wird.

6. Verfahren nach Anspruch 3 oder 4, wobei Schritt c) aus einem Schritt der Bestimmung der Menge an Alkalimetall oder Erdalkalimetall aus der Menge der in Schritt b) gesammelten Gase besteht und diese Gase dem im Reaktionsschritt gebildeten Dihydrogen und dem bzw. den Gasen entsprechen, die die inerte Atmosphäre des geschlossenen Behälters bilden, bevor dieser zur Ausführung von Schritt a) geöffnet wird.

7. Verfahren nach einem der vorhergehenden Ansprüche, wobei der Schritt des Sammelns von Dihydrogen und gegebenenfalls des bzw. der Gase, die die inerte Atmosphäre bilden und ursprünglich in dem geschlossenen Behälter enthalten sind, mit Hilfe eines Messzylinders (11) ausgeführt wird, wobei die so gesammelte Gasmenge durch Ablesen der auf dem Messzylinder aufgedruckten Skala in Volumen gemessen wird.

8. Verfahren nach Anspruch 7, wobei dann, wenn das Reagenz, das mindestens eine -OH-Funktion trägt, Wasser (3; 19) ist, der Schritt des Sammelns mittels eines Messzylinders ausgeführt wird, der ursprünglich vollständig mit Wasser gefüllt ist, wobei der Messzylinder mit dem offenen Ende nach unten senkrecht zu der Stelle, die von dem das Alkali- oder Erdalkalimetall enthaltenden Material eingenommen wird, in das flüssige Medium gestellt wird, in dem die Reaktion mit dem im Material enthaltenen Alkali- oder Erdalkalimetall stattfindet.

9. Verfahren nach einem der Ansprüche 1 bis 6, wobei der Schritt des Sammelns mittels eines Sammelbehälters ausgeführt wird, wobei die so gesammelte Gasmenge durch die Massendifferenz zwischen dem vor dem Sammeln ursprünglich vollständig mit Wasser gefüllten Sammelbehälter und dem Behälter, in dem ein Teil des ursprünglich im Behälter enthaltenen Wasservolumens durch das gesammelte Gasvolumen (15; 31) ersetzt ist, bestimmt wird.

10. Verfahren nach Anspruch 9, wobei dann, wenn das mindestens eine -OH-Funktion tragende Reagenz Wasser ist, der Schritt des Sammelns mittels eines zunächst mit Wasser gefüllten Sammelbehälters ausgeführt wird, der mit dem offenen Ende nach unten senkrecht zu der von dem das Alkali- oder Erdalkalimetall enthaltenden Material eingenommenen Stelle in das flüssige Medium gestellt wird, in dem die Reaktion mit dem im Material enthaltenen Alkali- oder Erdalkalimetall stattfindet.

11. Verfahren nach Anspruch 3, wobei dann, wenn der Schritt des Sammelns wie in Anspruch 8 definiert erfolgt, der Schritt der Bestimmung der Menge an Alkalimetall oder Erdalkalimetall aus der gesammelten Menge an Dihydrogen und Gas bzw. Gasen, die die inerte Atmosphäre des geschlossenen Behälters bilden, unter Anwendung der folgenden Gleichung (I) ausgeführt wird:

$$n_m = \frac{\frac{P_{Gas}}{T_{Gas}} \times V_{Gas} - \frac{P_{atm}}{T_{Wasser}} \times \frac{m_{Wasser}}{d_{Wasser}}}{a \times R - \frac{V_{mol\,m} \times P_{atm}}{T_{Wasser}}}$$

$$(I)$$

worin:

- $n_m$ der Menge an Alkali- oder Erdalkalimetall, die in dem Material enthalten ist, entspricht, wobei diese Menge in Mol ausgedrückt wird;
- $P_{Gas}$ dem Druck des in dem Messzylinder gesammelten Gases entspricht, wobei dieses Gas einem Gemisch aus dem Gas bzw. den Gasen, die die inerte Atmosphäre des geschlossenen Behälters bilden, und dem gesamten während der Reaktion erzeugten Dihydrogen entspricht, wobei der Druck in mmHg ausgedrückt wird;
- $P_{atm}$ dem atmosphärischen Druck entspricht, wobei dieser Druck in mmHg ausgedrückt wird;
- $T_{Gas}$ der Temperatur des im Messzylinder gesammelten Gases entspricht, wobei die Temperatur in K ausgedrückt wird;
- $V_{Gas}$ dem Volumen des im Messzylinder gesammelten Gases entspricht, wobei dieses Volumen dem des Gasgemisches entspricht, das das Gas bzw. die Gase, die die inerte Atmosphäre des geschlossenen Behälters bilden, und das gesamte während der Reaktion erzeugte Dihydrogen umfasst, wobei das Volumen in cm$^3$ ausgedrückt wird;
- $T_{Wasser}$ der Temperatur des im Messzylinder enthaltenen Wassers entspricht, wobei die Temperatur in K ausgedrückt wird;
- $m_{Wasser}$ der verdrängten Wassermenge entspricht, die der Differenz ($m_B$-$m_A$) entspricht, wobei $m_B$ die Gesamtmasse des geschlossenen Behälters bezeichnet, dessen gesamtes Volumen von Wasser eingenommen ist, und $m_A$ der Gesamtmasse des geschlossenen Behälters entspricht, der das Material und die inerte Atmosphäre enthält, wobei diese Masse in Gramm ausgedrückt wird;

- $d_{Wasser}$ der Dichte von Wasser entspricht;

- a dem dem Dihydrogen zugeordneten stöchiometrischen Koeffizienten in der Gleichung für die Bildung von Alkali- oder Erdalkalimetallhydroxid durch Wasser auf der Grundlage einer Gleichung, bei der der dem Metall zugeordnete stöchiometrische Koeffizient auf 1 reduziert ist, entspricht;

- R der Konstante der idealen Gase (d.h. 62359 mmHg.cm$^3$.°K$^{-1}$.mol$^{-1}$) entspricht;

- $V_{mol\ m}$ dem molaren Volumen von Alkali- oder Erdalkalimetall (ausgedrückt in cm$^3$/mol) entspricht.

12. Verfahren nach Anspruch 4, wobei dann, wenn der Schritt des Sammelns wie in Anspruch 8 definiert erfolgt, der Schritt der Bestimmung der Menge an Alkali- oder Erdalkalimetall aus der Menge an gesammelten Dihydrogen und gegebenenfalls Gas bzw. Gasen, die aus der inerten Atmosphäre des geschlossenen Behälters stammen, unter Anwendung der folgenden Gleichung (V) ausgeführt wird:

$$n_m = \frac{\frac{V_{Gas}}{T_{Gas}} \times P_{Gas}}{a \times R - \frac{V_{mol\ m} \times P_{atm}}{T_{Wasser}}}$$

$$(V)$$

worin:

- $n_m$ der Menge an Alkali- oder Erdalkalimetall, die in dem Material enthalten ist, entspricht, wobei diese Menge in Mol ausgedrückt wird;

- $P_{Gas}$ dem Druck des in dem Messzylinder gesammelten Gases entspricht, wobei dieses Gas in diesem Fall dem gesamten während der Reaktion erzeugten Dihydrogen und gegebenenfalls dem Gas bzw. den Gasen, die aus der inerten Atmosphäre des geschlossenen Behälters stammen, entspricht, wobei der Druck in mmHg ausgedrückt wird;

- $P_{atm}$ dem atmosphärischen Druck entspricht, wobei dieser Druck in mmHg ausgedrückt wird;

- $T_{Gas}$ der Temperatur des im Messzylinder gesammelten Gases entspricht, wobei die Temperatur in K ausgedrückt wird;

- $V_{Gas}$ dem Volumen des im Messzylinder gesammelten Gases entspricht, wobei dieses Volumen dem gesamten während der Reaktion erzeugten Dihydrogen und gegebenenfalls dem Gas bzw. den Gasen, die aus der inerten Atmosphäre des geschlossenen Behälters stammen, entspricht, wobei das Volumen in cm$^3$ ausgedrückt wird;

- a dem dem Dihydrogen zugeordneten stöchiometrischen Koeffizienten in der Gleichung für die Bildung von Alkali- oder Erdalkalimetallhydroxid durch Wasser auf der Grundlage einer Gleichung, bei der der dem Metall zugeordnete stöchiometrische Koeffizient auf 1 reduziert ist, entspricht;

- R der Konstante der idealen Gase (d.h. 62359 mmHg.cm$^3$.°K$^{-1}$.mol$^{-1}$) entspricht;

- $T_{Wasser}$ der Temperatur des im Messzylinder enthaltenen Wassers entspricht, wobei die Temperatur in K ausgedrückt wird;

- $V_{mol\ m}$ dem molaren Volumen des Metalls (ausgedrückt in cm$^3$/mol) entspricht.

13. Verfahren nach Anspruch 3, wobei dann, wenn der Schritt des Sammelns wie in Anspruch 10 definiert erfolgt, der Schritt der Bestimmung der Menge an Alkali- oder Erdalkalimetall aus der Menge an gesammeltem Dihydrogen und Gas bzw. Gasen aus der inerten Atmosphäre des geschlossenen Behälters unter Anwendung der folgenden Gleichung (VIII) ausgeführt wird:

$$n_m = \frac{\frac{(mc - md)}{T_{Gas}} \times P_{Gas} - \frac{P_{atm}}{T_{Wasser}} \times \frac{m_{Wasser}}{d_{Wasser}}}{a \times R - \frac{V_{mol\ m} \times P_{atm}}{T_{Wasser}}}$$

$$(VIII)$$

worin:

- $n_m$ der Menge an Alkali- oder Erdalkalimetall, die in dem Material enthalten ist, entspricht, wobei diese Menge in Mol ausgedrückt wird;
- $P_{Gas}$ dem Druck des in dem Sammelbehälter gesammelten Gases entspricht, wobei dieses Gas einem Gemisch aus dem Gas bzw. den Gasen, die aus der inerten Atmosphäre des geschlossenen Behälters stammen, und dem gesamten während der Reaktion erzeugten Dihydrogen entspricht, wobei der Druck in mmHg ausgedrückt wird;
- $P_{atm}$ dem atmosphärischen Druck entspricht, wobei dieser Druck in mmHg ausgedrückt wird;
- $T_{Gas}$ der Temperatur des im Sammelbehälter gesammelten Gases entspricht, wobei die Temperatur in K ausgedrückt wird;
- mc der Masse des vollständig mit Wasser gefüllten Sammelbehälters entspricht;
- md der Masse des Sammelbehälters nach dem Sammeln von Gas bzw. Gasen aus der inerten Atmosphäre des geschlossenen Behälters und des gesamten während der Reaktion erzeugten Dihydrogens entspricht;
- $T_{Wasser}$ der Temperatur des im Sammelbehälter enthaltenen Wassers entspricht, wobei die Temperatur in K ausgedrückt wird;
- $m_{Wasser}$ der verdrängten Wassermenge entspricht, die der Differenz ($m_B$-$m_A$) entspricht, wobei $m_B$ die Gesamtmasse des geschlossenen Behälters bezeichnet, dessen gesamtes Volumen von Wasser eingenommen wird, und $m_A$ der Gesamtmasse des geschlossenen Behälters entspricht, der das Material und die inerte Atmosphäre enthält;
- $d_{Wasser}$ der Dichte von Wasser entspricht;
- a dem dem Dihydrogen zugeordneten stöchiometrischen Koeffizienten in der Gleichung für die Bildung von Alkali- oder Erdalkalimetallhydroxid durch Wasser auf der Grundlage einer Gleichung, bei der der dem Metall zugeordnete stöchiometrische Koeffizient auf 1 reduziert ist, entspricht;
- R der Konstante der idealen Gase (d.h. 62359 mmHg.cm$^3$.°K$^{-1}$.mol$^{-1}$) entspricht;
- $V_{mol\ m}$ dem molaren Volumen des Metalls (ausgedrückt in cm$^3$/mol) entspricht.

14. Verfahren nach Anspruch 4, wobei dann, wenn der Schritt des Sammelns wie in Anspruch 10 definiert erfolgt, der Schritt der Bestimmung der Menge an Alkali- oder Erdalkalimetall aus der Menge an gesammeltem Dihydrogen und Gas bzw. Gasen aus der inerten Atmosphäre des geschlossenen Behälters unter Anwendung der folgenden Gleichung (XI) ausgeführt wird:

$$n_m = \frac{\frac{(mc-md)}{T_{Gas}} \times P_{Gas}}{a \times R - \frac{V_{mol\ m} \times P_{atm}}{T_{Wasser}}}$$

$$(XI)$$

worin:

- $n_m$ der Menge an Alkali- oder Erdalkalimetall, die in dem Material enthalten ist, entspricht, wobei diese Menge in Mol ausgedrückt wird;
- $P_{Gas}$ dem Druck des in dem Sammelbehälter gesammelten Gases entspricht, wobei dieses Gas dem gesamten während der Reaktion erzeugten Dihydrogen und gegebenenfalls dem Gas bzw. den Gasen, die aus der inerten Atmosphäre des geschlossenen Behälters stammen, entspricht, wobei der Druck in mmHg ausgedrückt wird;
- $P_{atm}$ dem atmosphärischen Druck entspricht, wobei dieser Druck in mmHg ausgedrückt wird;
- $T_{Gas}$ der Temperatur des im Sammelbehälter gesammelten Gases entspricht, wobei die Temperatur in K ausgedrückt wird;
- mc der Masse des vollständig mit Wasser gefüllten Sammelbehälters entspricht;
- md der Masse des Sammelbehälters nach dem Sammeln von dem inerten Gas und des gesamten während der Reaktion erzeugten Dihydrogens entspricht;
- $T_{Wasser}$ der Temperatur des im Sammelbehälter enthaltenen Wassers entspricht, wobei die Temperatur in K ausgedrückt wird;
- a dem dem Dihydrogen zugeordneten stöchiometrischen Koeffizienten in der Gleichung für die Bildung von Alkali- oder Erdalkalimetallhydroxid durch Wasser auf der Grundlage einer Gleichung, bei der der dem Metall zugeordnete stöchiometrische Koeffizient auf 1 reduziert ist, entspricht;
- R der Konstante der idealen Gase (d.h. 62359 mmHg.cm$^3$.°K$^{-1}$.mol$^{-1}$) entspricht;
- $V_{mol\ m}$ dem molaren Volumen des Metalls (ausgedrückt in cm$^3$/mol) entspricht.

**15.** Verfahren zur Bestimmung der Kapazität eines Alkali- oder Erdalkalimetalls, das in einem Material enthalten ist, umfassend die folgenden Schritte:

d) einen Schritt zur Durchführung des Verfahrens zur Bestimmung der molaren Menge eines Alkali- oder Erdalkalimetalls, das in einem Material enthalten ist, wobei das Verfahren nach einem der Ansprüche 1 bis 14 durchgeführt wird;

e) einen Schritt des Bestimmens der Molanzahl der in Schritt a) des Molmengenbestimmungsverfahrens ausgetauschten Elektronen ausgehend von der in d) erhaltenen Menge;

f) einen Schritt des Bestimmens der Kapazität in Amperestunden ausgehend von der in e) erhaltenen Elektronenmolanzahl.

**Claims**

**1.** A method for determining the quantity of alkali metal or alkaline earth metal present in a material (7; 23) comprising the following steps:

a) a step of quantitative reaction of the alkali metal or alkaline earth metal by contacting the latter with a liquid medium comprising a reagent carrying at least one -OH function, whereby dihydrogen and an alkali or alkaline earth metal hydroxide or oxide are formed;

b) a step of collecting the quantity of dihydrogen emitted during step a);

c) a step of determining the quantity of alkali metal or alkaline earth metal from the quantity of dihydrogen collected during step b).

**2.** The method according to claim 1, wherein the reagent carrying at least one -OH function is water (3; 19), an alcoholic compound and mixtures thereof.

**3.** The method according to claim 1 or 2, wherein, before the implementation of step a), the material comprising the alkali or alkaline earth metal is packaged in a closed container (5; 21) comprising an inert atmosphere (9; 25), which closed container is then opened for the implementation of step a).

**4.** The method according to any one of claims 1 to 2, wherein, before the implementation of step a), the material comprising the alkali or alkaline earth metal is packaged in a closed container under vacuum and comprising, optionally, partly, an inert atmosphere, which closed container is then opened for the implementation of step a).

**5.** The method according to claim 3 or 4, wherein step b) consists, in addition to collecting the dihydrogen resulting from step b), in collecting the constituent gas or gases from the inert atmosphere of the closed container before opening the latter for the implementation of step a).

**6.** The method according to claim 3 or 4, wherein step c) consists of a step of determining the quantity of alkali metal or alkaline earth metal from the quantity of gas collected during step b) and, these gases corresponding to the dihydrogen formed during the reaction step and to the constituent gas or gases of the inert atmosphere of the closed container before opening the latter for the implementation of step a).

**7.** The method according to any one of the preceding claims, wherein the step of collecting the dihydrogen and, where appropriate, the constituent gas or gases of the inert atmosphere initially contained in the closed container is carried out by means of a graduated test tube (11), the quantity of gas thus collected being measured in volume by reading the graduation printed on the test tube.

**8.** The method according to claim 7, wherein, when the reagent carrying at least one -OH function is water (3; 19), the collection step is carried out via a graduated test tube initially completely filled with water, which test tube is placed in the liquid medium, wherein the reaction takes place with the alkali or alkaline earth metal contained in the material, open end downwards, vertically to the place occupied by the material comprising the alkali or alkaline earth metal.

**9.** The method according to any one of claims 1 to 6, wherein the collection step is carried out by means of a collecting container, the quantity of gas thus collected being determined by mass difference between the collecting container which is initially completely filled with water before collection and the container, whereof part of the volume of water initially contained in the container is replaced by the volume of gas collected (15; 31).

10. The method according to claim 9, wherein, when the reagent carrying at least one -OH function is water, the collection step is carried out via a collecting container initially filled with water, which container is disposed in the liquid medium, wherein the reaction takes place with the alkali or alkaline earth metal contained in the material, open end downwards, vertically to the place occupied by the material comprising the alkali or alkaline earth metal.

11. The method according to claim 3, wherein, when the collection step is as defined in claim 8, the step of determining the quantity of alkali or alkaline earth metal from the collected quantity of dihydrogen and of the gas or gases constituting the inert atmosphere of the closed container is carried out using the following equation (I):

$$n_m = \frac{\dfrac{P_{gas}}{T_{gas}} \times V_{gas} - \dfrac{P_{atm}}{T_{water}} \times \dfrac{m_{water}}{d_{water}}}{a \times R - \dfrac{V_{mol\,m} \times P_{atm}}{T_{water}}}$$

(I)

wherein:

- $n_m$ corresponds to the quantity of alkali or alkaline earth metal contained in the material, said quantity being expressed in moles;
- $P_{gas}$ corresponds to the pressure of gas collected in the test tube, this gas corresponding to a mixture of the gas or gases constituting the inert atmosphere of the closed container and all the dihydrogen produced during the reaction, the pressure being expressed in mm of Hg;
- $P_{atm}$ corresponds to the atmospheric pressure, this pressure being expressed in mm of Hg;
- $T_{gas}$ corresponds to the temperature of the gas collected in the test tube, the temperature being expressed in K;
- $V_{gas}$ corresponds to the volume of gas collected in the test tube, this volume corresponding to that of the gas mixture comprising the gas or gases constituting the inert atmosphere of the closed container and all the dihydrogen produced during the reaction, the volume being expressed in $cm^3$;
- $T_{water}$ corresponds to the temperature of the water contained in the test tube, the temperature being expressed in K;
- $m_{water}$ corresponds to the quantity of water displaced corresponding to the difference ($m_B$-$m_A$), $m_B$ designating the total mass of the closed container having all its volume occupied by water and $m_A$ corresponds to the total mass of the closed container comprising the material and the inert atmosphere, this mass being expressed in grams;
- $d_{water}$ corresponds to the density of water;
- a corresponds to the stoichiometric coefficient assigned to dihydrogen in the equation for the formation of an alkali or alkaline earth metal hydroxide by water on the basis of an equation where the stoichiometric coefficient assigned to the metal is reduced to 1;
- R corresponds to the ideal gas constant (that is to say 62359 mm Hg.$cm^3$. ° $K^{-1}$.$mol^{-1}$);
- $V_{mol\,m}$ corresponds to the molar volume of the alkali or alkaline earth metal (expressed in $cm^3$/mol).

12. The method according to claim 4, wherein, when the collection step is as defined in claim 8, the step of determining the quantity of alkali or alkaline earth metal from the quantity of dihydrogen collected and, where appropriate, the gas or gases resulting from the inert atmosphere of the closed container, is carried out using the following equation (V):

$$n_m = \frac{\dfrac{P_{gas}}{T_{gas}} \times P_{gas}}{a \times R - \dfrac{V_{mol\,m} \times P_{atm}}{T_{water}}}$$

(V)

wherein:

- $n_m$ corresponds to the quantity of alkali or alkaline earth metal contained in the material, said quantity being expressed in moles;
- $P_{gas}$ corresponds to the pressure of gas collected in the test tube, this gas corresponding, in this case, to all the dihydrogen produced during the reaction and, where appropriate, to the gas or gases resulting from the inert atmosphere of the closed container, the pressure being expressed in mm of Hg;
- $P_{atm}$ corresponds to the atmospheric pressure, this pressure being expressed in mm of Hg;
- $T_{gas}$ corresponds to the temperature of the gas collected in the test tube, the temperature being expressed in K;
- $V_{gas}$ corresponds to the volume of gas collected in the test tube, this volume corresponding to all the dihydrogen produced during the reaction and, where appropriate, the gas or gases resulting from the inert atmosphere of the closed container, the volume being expressed in $cm^3$;
- a corresponds to the stoichiometric coefficient assigned to dihydrogen in the equation for the formation of an alkali or alkaline earth metal hydroxide by water on the basis of an equation where the stoichiometric coefficient assigned to the metal is reduced to 1;
- R corresponds to the ideal gas constant (that is to say 62359 mm Hg.$cm^3$.$°K^{-1}$.$mol^{-1}$);
- $T_{water}$ corresponds to the temperature of the water contained in the test tube, the temperature being expressed in K;
- $V_{mol\,m}$ corresponds to the molar volume of the metal (expressed in $cm^3/mol$).

13. The method according to claim 3, wherein when the collection step is as defined in claim 10, wherein the step of determining the quantity of alkali or alkaline earth metal from the quantity of dihydrogen collected and the gas or gases from the inert atmosphere of the closed container is carried out via the following equation (VIII):

$$n_m = \frac{\dfrac{(mc - md)}{T_{gas}} \times P_{gas} - \dfrac{P_{atm}}{T_{water}} \times \dfrac{m_{water}}{d_{water}}}{a \times R - \dfrac{V_{mol\,m} \times P_{atm}}{T_{water}}}$$

(VIII)

wherein:

- $n_m$ corresponds to the quantity of alkali or alkaline earth metal contained in the material, said quantity being expressed in moles;
- $P_{gas}$ corresponds to the pressure of the gas collected in the collecting container, this gas corresponding to a mixture of the gas or gases resulting from the inert atmosphere of the closed container and all the dihydrogen produced during the reaction, the pressure being expressed in mm of Hg;
- $P_{atm}$ corresponds to the atmospheric pressure, this pressure being expressed in mm of Hg;
- $T_{gas}$ corresponds to the temperature of the gas collected in the collecting container, the temperature being expressed in K;
- mc corresponds to the mass of the collecting container completely filled with water;
- md corresponds to the mass of the collecting container after collection of the gas or gases resulting from the inert atmosphere of the closed container and all the dihydrogen produced at the end of the reaction;
- $T_{water}$ corresponds to the temperature of the water contained in the collecting container, the temperature being expressed in K;
- $m_{water}$ corresponds to the quantity of water displaced corresponding to the difference ($m_B$-$m_A$), $m_B$ designating the total mass of the closed container having all its volume occupied by water and $m_A$ corresponds to the total mass of the closed container comprising the material and the inert atmosphere;
- $d_{water}$ corresponds to the density of water;
- a corresponds to the stoichiometric coefficient assigned to dihydrogen in the equation for the formation of the alkali or alkaline earth metal hydroxide by water on the basis of an equation where the stoichiometric coefficient assigned to the metal is reduced to 1;
- R corresponds to the ideal gas constant (that is to say 62359 mm Hg.$cm^3$.$°K^{-1}$.$mol^{-1}$);
- $V_{mol\,m}$ corresponds to the molar volume of the metal (expressed in $cm^3/mol$).

14. The method according to claim 4, wherein, when the collection step is as defined in claim 10, the step of determining the quantity of alkali or alkaline earth metal from the quantity of dihydrogen collected and, where appropriate, the gas or gases resulting from the inert atmosphere of the closed container, is carried out using the following equation (XI):

$$n_m = \frac{\dfrac{(mc - md)}{T_{gas}} \times P_{gas}}{a \times R - \dfrac{V_{mol\ m} \times P_{atm}}{T_{water}}}$$

(XI)

wherein:

- $n_m$ corresponds to the quantity of alkali or alkaline earth metal contained in the material, said quantity being expressed in moles;
- $P_{gas}$ corresponds to the pressure of gas collected in the collecting container, this gas corresponding to all the dihydrogen produced during the reaction and, where appropriate, to the gas or gases resulting from the inert atmosphere of the closed container, the pressure being expressed in mm of Hg;
- $P_{atm}$ corresponds to the atmospheric pressure, this pressure being expressed in mm of Hg;
- $T_{gas}$ corresponds to the temperature of the gas collected in the collecting container, the temperature being expressed in K;
- mc corresponds to the mass of the collecting container completely filled with water;
- md corresponds to the mass of the collecting container after collection of the inert gas and all the dihydrogen produced at the end of the reaction;
- $T_{water}$ corresponds to the temperature of the water contained in the collecting container, the temperature being expressed in K;
- a corresponds to the stoichiometric coefficient assigned to dihydrogen in the equation for the formation of alkali or alkaline earth metal hydroxide by water on the basis of an equation where the stoichiometric coefficient assigned to the metal is reduced to 1;
- R corresponds to the ideal gas constant (that is to say 62359 mm Hg.cm$^3$. ° K$^{-1}$.mol$^{-1}$);
- $V_{mol\ m}$ corresponds to the molar volume of the metal (expressed in cm$^3$/mol).

15. A method for determining the capacity of an alkali or alkaline earth metal contained in a material comprising the following steps:

   d) a step of implementing the method for determining the molar quantity of an alkali or alkaline earth metal contained in a material, the method being as defined according to any one of claims 1 to 14;
   e) from the quantity obtained in d), a step of determining the number of moles of electrons exchanged during step a) of the method for determining the molar quantity;
   f) from the number of moles of electrons obtained in e), a step of determining the capacity in ampere-hours,

FIG. 1

FIG. 2

FIG. 3

FIG. 4

## RÉFÉRENCES CITÉES DANS LA DESCRIPTION

**Documents brevets cités dans la description**

- CN 203772732 U

**Littérature non-brevet citée dans la description**

- *Journal of the less-common Metals,* 1981, vol. 80, 241-255

- *Anal. Chem.,* 1968, vol. 40 (11), 1731-1732